# EUROPÄISCHE PATENTSCHRIFT

(11) **EP 2 482 766 B1**
(45) Veröffentlichungstag und Bekanntmachung des Hinweises auf die Patenterteilung: **25.02.2015**
(21) Anmeldenummer: 10759650.4
(22) Anmeldetag: 29.09.2010
(51) Int. Cl.: A61F 5/11, A61Q 3/02, A61K 8/81

(54) **KORREKTURVERFAHREN FÜR HAND- ODER FUSSNÄGEL**
CORRECTIVE TREATMENT OF FINGER- OR TOENAILS
PROCÉDÉ DE CORRECTION POUR ONGLES DES MAINS OU DES PIEDS

(30) Priorität: 29.09.2009 DE 102009043355
(43) Veröffentlichungstag der Anmeldung: 08.08.2012
(73) Patentinhaber: Hillebrand, Christa, 83700 Rottach-Weissach (DE)
(72) Erfinder: Hillebrand, Christa, 83700 Rottach-Weissach (DE)
(74) Vertreter: Franke, Dirk
(86) Internationale Anmeldenummer: PCT/EP2010/064439
(87) Internationale Veröffentlichungsnummer: WO 2011/039243

(56) Entgegenhaltungen:
- US-A- 5 261 872
- US-A1- 2004 156 801
- US-A1- 2008 287 912

## Beschreibung

Die Erfindung betrifft ein Verfahren zur Korrektur eines Hand- oder Fußnagels umfassend das Auftragen mindestens einer Schicht und das Durchführen mindestens eines Behandlungsschritts, in welchem sich die mindestens eine Schicht kontrahiert oder expandiert.

Traditionell werden zur Korrektur von eingewachsenen oder eingerollten Hand- oder Fußnägeln Nagelkorrekturspangen verwendet. Hierzu werden Gipsmodelle des zu korrigierenden Nagels angefertigt und exakt nach diesen Modellen federnde Spangen hergestellt, welche hakenartige Enden aufweisen, die um den Rand des Nagels greifen und als Befestigungseinrichtungen für die Spange dienen. Diese Art der Nagelkorrektur ist zwar in der Regel wirkungsvoll, weist jedoch den Nachteil auf, dass die Anfertigung von Gipsmodellen und entsprechend dimensionierter Spangen aufwendig ist. Zudem finden die hakenartigen Enden der Spangen an solchen Nägeln oft keinen festen Halt, so dass sie seitlich ausbrechen können und es zum Wachstum von sogenanntem wildem Fleisch kommt.

Um die Anfertigung spezieller Spangen für die Nagelkorrektur zu vermeiden, ist es auch bekannt, am Nagel Plättchen mit offenen Ausnehmungen zu befestigen, und in diese Ausnehmungen die Enden von zwei mit je einem seitlichen Haken den Nagel umgreifenden Spangenenden einzurasten.

So wird in der Offenlegungsschrift DE 10 2007 035 031 A1 eine Vorrichtung zur Korrektur der Wuchsform von menschlichen Nägeln offenbart, die ein Haftelement umfasst, welches durch Klebstoff auf einem Teilbereich der Nagelplatte einer menschlichen Zehe fixiert wird. Dabei wird mit einem in Teilbereichen fest in das Haftelement eingebetteten Zugelementen eine Kraft ausgeübt, welche durch außermittigen Kraftangriff ein Biegemoment auf die Nagelplatte überträgt und die Nagelränder anhebt.

Die Offenlegungsschrift DE 33 30 813 A1 offenbart eine Vorrichtung zur Vornahme von Nagelkorrekturen, umfassend eine Befestigungseinrichtungen mit dem zu korrigierenden Nagel verbundene Federanordnung.

Die Offenlegungsschrift DE 42 07 797 A1 offenbart eine Nagelkorrekturspange, bestehen aus zwei oder mehreren Spangenteilen aus Draht, welche seitlich über einem Teil des Nagels liegen und welche an einem Ende mit einem unter den Nagel greifenden Haken versehen sind.

Die Offenlegungsschrift DE 32 36 804 A1 offenbart eine Nagelkorrekturspange aus Federstahlband in einer der jeweiligen Breite eines Fußnagels erforderlichen Länge, bei welchem an jedem Ende ein Gummiprofil aufgeschoben wird.

Nachteilig bei diesen Verfahren des Standes der Technik ist jedoch, dass auch bei diesen Befestigungseinrichtungen in der Regel Klammern oder ähnliches verwendet werden, die zu schmerzhaften Druckstellen in der Nähe des Hand- bzw. Fußnagels führen können. Darüber hinaus können die Haken bei längerer Tragezeit auch zu schweren Entzündungen führen, welche unbehandelt bis zum vollständigen Verlust des Nagels führen können.

Der Erfindung liegt deshalb die Aufgabe zugrunde, ein Verfahren bereitzustellen, welches die oben genannten Nachteile des Standes der Technik nicht aufweist. Das Verfahren soll universell einsetzbar sein und unabhängig von der Ausbildung des seitlichen Nagelrandes bzw. des Nagelbetts in diesem Bereich ohne Beschwerden für den Benutzer angewendet werden können.

Erfindungsgemäß wurde das Problem durch das Verfahren gemäß den beiliegenden Patentansprüchen gelöst, mit welchem korrigierte Hand- oder Fußnägel hergestellt werden können.

Beispielsweise kann das Verfahren ein kosmetisches Verfahren sein, wenn beispielsweise der Nagel eine unerwünscht hohe konvexe Krümmung aufweist, die korrigiert werden soll. Das erfindungsgemäße Verfahren ist schnell und einfach anwendbar, so dass es hohen Effizienzanforderungen in der kosmetischen Praxis gut gerecht wird. Im Vergleich zu den Verfahren des Standes der Technik ist das erfindungsgemäße Verfahren im Wesentlichen schmerzfrei, da auf metallische Korrekturhilfen verzichtet werden kann. Die Auftragung der Schichten erfolgt ohne Eingriff in den Körper, so dass operative Belastungen weitgehend vermieden werden.

Die Hand- oder Fußnägel bewirken aufgrund der durch das erfindungsgemäße Verfahren erzeugten Planarität einen ansprechenden ästhetischen Effekt.

Das erfindungsgemäße Verfahren ermöglicht einen völligen Verzicht auf eine Hakenbefestigung am Nagelrand und darüber hinaus kann es für völlig unterschiedliche, zu behandelnde Nägel angewendet werden.

Überdies kann das Verfahren auch ein therapeutisches Verfahren sein, wenn eine krankhafte Nagelveränderung, wie beispielsweise ein krankhafter Rollnagel, behandelt werden soll. In diesem Fall ist das Verfahren geeignet, den hohen Effizienzanforderungen in der ärztlichen und podologischen Praxis gerecht zu werden. Durch Anwendung des Verfahrens können Nagelbettinfektionen wirkungsvoll verhindert werden.

Weitere besondere Ausführungsformen und vorteilhafte Effekte sind in dieser Offenbarung beschrieben.

### [Detaillierte Beschreibung der Erfindung]

In einem ersten Aspekt betrifft die Erfindung ein Verfahren zur Korrektur eines Hand- oder Fußnagels (2). Das Verfahren umfasst folgende Schritte. In einem ersten Schritt a) wird mindestens eine Schicht (3,4) mindestens einer Zusammensetzung auf einen Hand- oder Fußnagel aufgetragen. In einem zweiten Schritt b) wird mindestens ein Behandlungsschritt durchgeführt, in welchem sich mindestens eine der aufgetragenen Schichten (3,4) kontrahiert oder expandiert. Hierdurch wird der Wölbungsgrad des Hand- oder Fußnagels (2) geändert und der Hand- oder Fußnagel korrigiert.

Erfindungsgemäß wird unter Hand- oder Fußnagel jeder Nagel einer menschlichen oder tierischen Hand oder eines menschlichen oder tierischen Fußes verstanden. Vorzugsweise ist der Hand- oder Fußnagel ein menschlicher Hand- oder Fußnagel. Insbesondere betrifft die Erfindung ein Verfahren zur kosmetischen Korrektur eines menschlichen Fußnagels. Der Nagel hat in diesem Fall vorzugsweise eine unerwünscht hohe konvexe Krümmung, die korrigiert werden soll. Alternativ kann die Erfindung auch ein therapeutisches Verfahren betreffen, wenn eine krankhafte Nagelveränderung, wie beispielsweise ein krankhafter Rollnagel, behandelt werden soll, welche bei dem Patienten bereits häufig zu Entzündungen des Nagelfleisches bzw. des Nagelbetts geführt hat. Unter Korrektur wird im Sinne dieser Offenbarung jeder Verfahrensschritt verstanden, der die unerwünschte Wölbung des Hand- oder Fußnagels korrigieren kann. Beispielsweise wird unter Korrektur die Verringerung der Konvexität eines Rollnagels verstanden, so dass der Nagel nach der Korrektur im Wesentlichen planar ist oder zumindest geringere Konvexität aufweist.

In dem ersten Schritt a) wird mindestens eine Schicht (3,4) mindestens einer Zusammensetzung auf den Hand- oder Fußnagel aufgetragen. Dies bedeutet mit anderen Worten, dass die Nagelplatte vollständig oder zumindest teilweise mit der aus mindestens einer Zusammensetzung bestehenden Schicht bedeckt wird. Das Auftragen selbst kann durch herkömmliche mechanische Auftrageverfahren erfolgen, beispielsweise durch Eintauchen des Hand- oder Fußnagels in eine flüssige Zusammensetzung, durch schichtweises Aufpinseln der Zusammensetzung oder durch Sprühbeschichten. Vorzugsweise wird die mindestens eine Schicht mit einem Pinsel auf die vollständige Nagelplatte aufgetragen. Die Auftragung kann vorzugsweise auch in mehreren Schritten bzw. Stufen erfolgen, um eine sehr gleichmäßige, d.h. flächige Verteilung der Zusammensetzung auf der Nagelplatte zu erreichen. Vor dem Auftragen kann der Nagel erweicht oder verdünnt werden sowie erforderlichenfalls beschnitten werden. Besonders bevorzugt ist es, wenn die Nagelplatte vor dem Auftragen mechanisch aufgeraut wird, da dies zu einer sehr festen Verbindung zwischen der mindestens einen Schicht und der Nagelplatte führt.

Die Schicht umfasst mindestens eine Zusammensetzung, welche vorzugsweise ein Polymer oder eine Polymermischung ist, d.h. eine Zusammensetzung, welche mindestens eine Art von Polymeren aufweist. Die Zusammensetzung kann grundsätzlich jede beliebige Zusammensetzung sein, solange diese zur Auftragung auf einen Hand- oder Fußnagel geeignet ist und mit deren Hilfe die Wölbung eines Hand- oder Fußnagels korrigieren werden kann. Vorzugsweise ist die Zusammensetzung eine in einem wässrigen oder organischen Lösungsmittel gelöste vollständig oder teilweise polymerisierte oder polymerisierbare organische Verbindung. Besonders bevorzugt ist es, wenn die Zusammensetzung eine copolymerisierbare oder pfropfpolymerisierbare Oligomer- oder Polymermischung enthält. Vorzugsweise kann die Zusammensetzung mindestens eine photopolymerisierbare Verbindung auf der Basis von Acrylsäureestern enthalten. Als Polymer kann jedes Polymer verwendet werden, solange dieses sich auf dem Nagelbett verfestigen lässt.

Die Zusammensetzung kann beispielsweise eine hitzehärtbare Bindemittelmischung sein, die ein organisches Harz und ein Vernetzungsmittel auf der Basis von Michael-Addukten aus CH-aciden, enolisierbaren Carbonsäureestern mit alpha-betaethylenisch ungesättigten Molekülgruppen enthalten. Alternativ kann die Zusammensetzung auch ein Zwei-Komponentenlack sein, welcher in der Wärme härtbar und gleichzeitig expandierbar oder kontrahierbar ist, wobei die Härtungsreaktion durch Lewis- und/oder Brönstedt-Basen katalysiert wird.

Die Zusammensetzung kann Esteracrylate oder Estermethacrylate oder deren Gemische enthalten. Esteracrylate oder Estermethacrylate sind beispielsweise durch Umsetzung von Acrylsäure oder Methacrylsäure mit Verbindungen mit mindestens einer OH-Gruppe oder mindestens einer Epoxygruppe unter Kondensation oder Addition erhältlich. Als Verbindungen mit mindestens einer OH-Gruppe eignen sich grundsätzlich beliebige mono-oder polyfunktionelle Alkohole, vorzugsweise Alkohole mit zwei oder mehr, beispielsweise 2 bis 6, insbesondere 2 oder 3 OH-Gruppen. Geeignete Alkohole können sowohl aliphatischer als auch aromatischer Natur sein oder können beide Bausteine beinhalten. Geeignet sind allgemein ein- und mehrfunktionelle (Meth)acrylatmonomere, wobei der Begriff (Meth)acrylat sowohl für Verbindungen auf Basis von Acrylsäure als auch für Verbindungen auf Basis von Methacrylsäure steht. Typische Vertreter dieser Verbindungsklasse (beispielsweise beschrieben in der DE-A-43 28 960) sind Alkyl (meth)acrylate, einschließlich der Cycloalkyl(meth)acrylate, Aralkyl(meth)acrylate und 2-Hydroxyalkyl(meth)acrylate, beispielsweise Hydroxypropylmethacrylat, Hydroxyethylmethacrylat, Isobornylacrylat, Isobornylmethacrylat, Butylglycolmethacrylat, Acetylglykolmethacrylat, Triethylenglycoldimethacrylat, Polyethylenglycoldimethacrylat, 2-Phenylethylmethacrylat, 2-Ethylhexylmethacrylat, Cyclohexylmethacrylat, Laurylmethacrylat und Hexandioldi(meth)acrylat. Verwendet werden können auch langkettige Monomere auf der Basis von Bisphenol A und Glycidylmethacrylat, die aus der US-A-3 066 112 bekannt sind, oder deren durch Addition von Isocyanaten entstandenen Derivate (Urethanmethacrylate). Geeignet sind auch Verbindungen des Typs Bisphenol-A-diethyloxy(meth)-acrylat und Bisphenol-A-dipropyloxy(meth)acrylat. Weiterhin Verwendung finden können die oligoethoxylierten und oligopropoxylierten Bisphenol-A-diacryl- und -dimethacrylsäureester. Gut geeignet sind außerdem die in der DE-C-28 16 823 genannten Diacryl- und Dimethacrylsäureester des Bis(hydroxymethyl)-tricyclo[5.2.1.026]-decans und die Diacryl- und Di-methacrylsäureester der mit 1 bis 3 Ethylenoxid- und/oder Propylenoxideinheiten verlängerten Verbindungen des Bis(hydroxymethyl)-tricyclo[5.2.1.026]-decans. Die Herstellung entsprechender Esteracrylate oder Estermethacrylate ist dem Fachmann allgemeinen bekannt.

Die Zusammensetzung kann auch Urethanmethacrylate oder mehrfunktionellen Urethanacrylate enthalten. Urethanmethacrylate sind durch Umsetzung von Polyisocyanaten mit OH-funktionellen Acrylaten oder Methacrylaten wie Hydroxyethylacrylat, Hydroxypropylacrylat Hydroxyethylmethacrylat oder Hydroxypropylmethacrylat erhältlich. Wird als Polyisocyanat ein Diisocyanat eingesetzt, so erhält man als Produkt ein Urethandimethacrylat, bei Einsatz eines OH-funktionellen Acrylats erhält man analog ein difunktionelles Acrylat. Urethanmethacrylate oder Urethanacrylate weisen ausgezeichnete Eigenschaften, wie hohe Steifigkeit auf. Möglich ist auch der Einsatz eines aus einer Kombination von Triisocyanaten oder höherwertigeren Polyisocyanaten mit OH-funktionellen Acrylaten oder Methacrylaten hergestellten Monomeren, wobei derartige Urethanmethacrylate bzw. Urethanacrylate im Hinblick auf olefinisch ungesättigte Doppelbindungen eine Funktionalität von drei oder höher aufweisen.

Eine zur Herstellung entsprechender Polymerer geeignete Zusammensetzung kann über die oben genannten Bestandteile hinaus noch einen oder mehrere Reaktionsverdünner enthalten. Unter dem Begriff Reaktionsverdünner werden dabei im Rahmen der vorliegenden Offenbarung alle Verbindungen verstanden, die für die zur Polykondensation eingesetzte Masse als Lösungs- oder Verdünnungsmittel wirken und somit die Viskosität der Masse verringern, die jedoch darüber hinaus bei der Polymerisation der Masse, beispielsweise bei der Polymerisation mit Hilfe von Licht oder Wärme, in die Polymermatrix einpolymerisiert werden. Dazu geeignet sind beispielsweise weitere Monomethacrylate, Dimethacrylate und Trimethacrylate. So eignen sich beispielsweise Dimethacrylatcomonomere wie Triethylenglykoldimethacrylat ("TEGDM"), Ethylenglykoldimethacrylat, Tetramethylenglykoldimethacrylat, Trimethylolpropyltrimethacrylat, 1,6-Hexandioldimethacrylat und 1,3-Butandioldimethacrylat als zusätzliche Bestandteile der zu polymerisierenden Massen.

Weiterhin kann eine zur Polymerisation eingesetzte Polymermasse weitere Zusatzstoffe wie Füllstoffe, Photoinitiatorsysteme und Farbpigmente enthalten.

Die Zusammensetzung kann auch aus A) 10 bis 90 Gew.-% einer oder mehrerer als Vernetzer wirkenden Verbindungen mit im Mittel mindestens zwei CH-aciden Wasserstoffatomen und die aus einer oder mehreren der folgenden Gruppierungen stammen, die gleich oder verschieden sein können: worin -CN oder -NO₂ oder -CN, -H, Alkyl oder Alkylen, wobei die Reste jeweils über das Kohlenstoffatom an die CH-Gruppe gebunden sind und die CH-Gruppe über mindestens einen der Reste an eine polymere oder oligomere Einheit gebunden ist, B) 90 bis 10 Gew.-% eines oder mehrerer zur Michael-Addition geeigneter (Meth)acrylcopolymerer, Polyester und/oder Polyurethanharze mit mindestens zwei über das Carbonyl-Kohlenstoffatom von gebundenen, alpha-beta-ungesättigten Gruppen, und C) 0,01 bis 5 Gew.-%, bezogen auf die Summe der Gewichte der Komponenten A) und B) eines Katalysators in Form einer Lewis- oder Brönstedt-Base bestehen, wobei die konjugierten Säuren der letzteren einen pK_{A}-Wert von mindestens 10 haben.

Die Zusammensetzung kann auch ein Polyurethan oder eine Polyurethanmischung enthalten. Vorzugsweise ist das Polyurethan ausgewählt aus hydrophilen, wasserlöslichen oder wasserdispergierbaren Polyurethanen mit ionogenen oder ionischen Gruppen. Als Polyurethan kann beispielsweise verwendet werden: ein Polyurethan, welches aus einer Polyolkomponente gebildet ist, bei der es sich um ein Alkylenglycol, ein Polyoxyalkylenglycol oder um ein lineares Polyesterdiol handeln kann, einer Carbonsäureesterkomponente mit Hydroxy- oder Aminogruppen und einem organischen Isocyanat oder Isocyanatvorläufer, wobei die nach Polymerisation an das Polymerrückgrat gebundenen Estergruppen verseift sind; ein Polyurethane, aufgebaut aus a) mindestens einer Verbindung, die zwei oder mehrere aktive Wasserstoffe pro Molekül enthalten, b) mindestens einem Säure- oder Salzgruppen enthaltenden Diol und c) mindestens einem Diisocyanat; ein Polyurethane, aufgebaut aus a) mindestens einem Diisocyanat, welches bereits vorher mit einer oder mehreren Verbindungen, die zwei oder mehrere aktive Wasserstoffatome pro Molekül enthalten, umgesetzt sein kann und b) mindestens einem Diol, einem primären oder sekundären Aminoalkohol, einem primären oder sekundären Diamin oder einem primären oder sekundären Triamin mit einem oder mehreren tertiären, quartären oder protonierten tertiären Aminstickstoffatomen; ein Polyurethane mit Carboxylatgruppen und einem Gehalt an Monomer-Diolen der Formel A-C(CH₂OH)₂-COOH worin A für ein Wasserstoffatom oder eine C₁- bis C₂₀-Alkylgruppe steht; ein Polyurethane, gebildet aus a) einem in Wasser löslichen oder dispergierbaren Polyurethanpräpolymer mit endständigen Isocyanatgruppen und b) mindestens einem primären oder sekundären Amin, das wenigstens eine ionogene bzw. ionische Gruppe aufweist. Als Diisocyanat können beispielsweise Hexamethylendiisocyanat, 2,4- und 2,6-Toluoldiisocyanat, 4,4'-Methylendi(phenylisocyanat) oder Isophorondiisocyanat verwendet werden. Alternativ kann das Polymer auch natürlichen Ursprungs sein. Als Glykolkomponente kann eine Verbindung ausgewählt sein, welche aus Ethylenglykol, die Propylen- und Butylenglykole, Di-, Tri-, Tetra- und Polyethylen- und -propylenglykole, Copolymere von Alkylenoxiden wie Ethylenoxid, Propylenoxid oder Butylenoxid, Ethylendiamin, Propylendiamin, 1,4-Diaminobutan, Hexamethylendiamin und alpha-, omega-Diaminen von langkettigen Alkanen oder Polyalkylenoxiden ausgewählt ist. Die Zusammensetzung kann zusätzlich auch ein oder mehrere natürliche Polymer enthalten. Beispielsweise kann die Zusammensetzung auch ein natürliches Polymer enthalten, welches aus Schellack, Chitosan, alkoxylierten Chitosanen, alkoxylierten Chitinen, Polysacchariden und chinesischem Balsamharz ausgewählt ist.

Die Zusammensetzung kann einen oder mehrere Photoinitiatoren enthalten, die durch Bestrahlung mit UV- oder sichtbarem Licht die Polymerisationsreaktion auslösen können. Als Photoinitiatoren kann eine zur Polymerisation vorgesehene Polymermasse beispielsweise einzelne geeignete Photoinitiatoren enthalten. Es ist jedoch ebenso möglich, dass eine zur Polymerisation eingesetzte Polymermasse auch Initiatorsysteme enthält, die Photoinitiatoren und Co- Initiatoren umfassen. Vertreter solcher Photoinitiatoren sind beispielsweise Benzoinalkylether, Benzilketale, Acylphosphinoxide oder aliphatische und aromatische 1,2-Diketonverbindungen, beispielsweise Campherchinon, wobei die Lichtpolymerisation durch Zusatz von Aktivatoren, wie tertiären Aminen oder organischen Phosphiten, in an sich bekannter Weise beschleunigt werden kann. Geeignete Initiatorsysteme zur Auslösung der Polymerisation über einen Redox-Mechanismus sind beispielsweise die Systeme Peroxid/Amin oder Peroxid/Barbitursäurederivate. Bei Verwendung solcher Initiatorsysteme ist es zweckmäßig, einen Initiator (z. B. Peroxid) und eine Katalysatorkomponente (z. B. Amin) getrennt bereitzuhalten. Die beiden Komponenten werden dann kurz vor ihrer Anwendung miteinander homogen vermischt.

Die Zusammensetzung kann in einem oder in mehreren organischen Lösungsmitteln gelöst sein, welche vorzugsweise mit Wasser mischbar sind. Beispielsweise können Ethanol, Propanol, Isopropanol, Aceton, Methylethylketon, Essigsäuremethylester oder Essigsäureethylester oder Gemische aus zwei oder mehr davon als Lösungsmittel eingesetzt sein.

Vorzugsweise ist die mindestens eine Zusammensetzung im Wesentlichen flüssig, d.h. dünnflüssig, viskos oder zähflüssig, oder im Wesentlichen gelartig. Auf diese Weise kann die Zusammensetzung besser auf dem Nagel aufgetragen werden. Die Zusammensetzung sollte nach dem Auftragen erhärtbar, d.h. verfestigbar, sein, um eine im Wesentlichen feste Schicht zu ergeben. In einer Implementierung wird nach dem Auftragen die Verfestigung der Schicht dadurch erreicht, dass sich Lösungsmittel verflüchtigt und eine verfestigte Schicht zurücklässt. Das Verflüchtigen des Lösungsmittels kann bei Raumtemperatur durchgeführt werden oder die Schichten können zusätzlich erwärmt werden, beispielsweise durch Verwendung eines Heißluftföns, insbesondere bei Temperaturen im Bereich von 40 bis 60 °C. In einer anderen Implementierung kann die Schicht dadurch verfestigt werden, dass sich in der Zusammensetzung enthaltene Mono-, Oligo- oder Polymere vernetzen oder polymerisieren. Die vernetzten Polymerisate sorgen in diesem Falle für die Festigkeit der zurückbleibenden Schicht.

In dem zweiten Schritt b) wird mindestens ein Behandlungsschritt durchgeführt, wobei sich die mindestens eine Schicht (3,4) entweder kontrahiert, d.h. zusammenzieht, oder expandiert, d.h. ausdehnt. Hierdurch wird der Wölbungsgrad, d.h. der Konvexitätsgrad des Hand- oder Fußnagels (2), geändert und so der Hand- oder Fußnagel korrigiert. In einer besonders bevorzugten Implementierung kontrahiert sich die Schicht während der Verfestigung. Aufgrund der Zugspannung während der Kontraktion und der festen Verbindung zur Nagelplatte wird diese an den Enden angehoben, so dass der Hand- oder Fußnagel nach dem Schritt b) eine geringere Wölbung aufweist und dadurch korrigiert ist.

Die Dicke der aufgetragenen Schicht kann beliebig sein, solange diese geeignet ist, den Hand- oder Fußnagel zu korrigieren. Vorzugsweise beträgt die Dicke der aufgetragenen Schicht 100 µm bis 1 mm, besonders bevorzugt 200 µm bis 800 µm, insbesondere 300 µm bis 500 µm.

In einer bevorzugten Implementierung ist der mindestens eine Behandlungsschritt in Schritt b) ein Licht- oder Wärmebehandlungsschritt.

Beispielsweise kann sich die mindestens eine Schicht (3,4) aufgrund eines Wärmebehandlungsschritts aufgrund chemischer oder physikalischer Änderungen innerhalb der intrinsischen Struktur der Schicht kontrahieren. Beispielsweise kann der Hand- oder Fußnagel einen von der mindestens einen Schicht verschiedenen thermischen Expansionskoeffizienten aufweisen, so dass sich der Hand- oder Fußnagel infolge der thermischen Expansion bzw. der thermischen Kontraktion entgegen der Wölbung des Hand- oder Fußnagels in Richtung geringerer Konvexität biegt. Wenn die in Schritt a) aufgetragene Zusammensetzung ein vernetzbares Oligo- oder Polymer enthält, kann durch die Wärmebehandlung auch eine zweite Polymerisationsreaktion initiiert werden, das heißt gestartet werden, die zu einem Polymerisat oder Copolymerisat mit einem geringeren Raumvolumen führt, so dass aufgrund des Polymerisationsprozesses die Zugspannung in Richtung der Mitte des Hand- oder Fußnagels bewirkt wird.

In einer alternativen Implementierung ist der Behandlungsschritt ein Lichtbehandlungsschritt, d.h. eine Behandlung durch Aufstrahlen von Licht einer bestimmten Wellenlänge. Beispielsweise kann die Lichtbehandlung mit ultraviolettem (UV-)Licht mit einer Wellenlänge von unter 400 nm oder unterhalb von 315 nm oder unterhalb von 280 nm erfolgen. Besonders bevorzugt ist die Verwendung von blauem Licht im Bereich von 435 bis 495 nm, da dieses weniger bedenklich im Hinblick auf eine krebserzeugende Wirkung bei längerer oder intensiver Bestrahlung und somit aus medizinischen Gründen zu bevorzugen ist. Durch die Bestrahlung mit Licht einer bestimmten Wellenlänge ändert sich die räumliche oder chemische Struktur innerhalb der Schicht derart, dass sich die Schicht selbst kontrahiert oder expandiert, vorzugweise kontrahiert. Wenn die in Schritt a) aufgetragene Zusammensetzung vernetzbare Oligo- oder Polymere enthält, dann können durch die Bestrahlung mit UV-Licht beispielsweise Radikale erzeugt werden, welche Initiatoren einer radikalischen Polymerisation oder Copolymerisation sein können. Wenn die Schicht sich während einer solchen Polymerisation oder Copolymerisation kontrahiert, kann eine Nagelkorrektur erreicht werden.

Alternativ kann durch die Licht- oder Wärmebehandlung auch ein Phasenübergang in der mindestens einen Schicht hervorgerufen werden, der mit einer Expansion oder Kontraktion der mindestens einen Schicht einhergeht. Wenn beispielsweise die mindestens eine aufgetragene Schicht (3,4) ein amorpher Thermoplast ist, dann kann durch Erwärmen über die Glasübergangstemperatur T_{G} ein Phasenübergang eintreten, wobei die Schicht in eine Phase mit geringerem oder höherem Raumvolumen übergeht. Hierdurch wird der Nagel in Richtung geringerer Konvexität gebogen und somit korrigiert.

In einer weiteren Implementierung ist der mindestens eine Behandlungsschritt ein Kältebehandlungsschritt. Durch die Kältebehandlung kann ein Kunststoff, beispielsweise ein Elastomer, unterhalb der Glasübergangstemperatur in eine kristalline Phase übergehen, welche ein geringeres oder ein höheres Raumvolumen einnimmt. Hierdurch wird die Korrektur des Hand- bzw. Fußnagels erreicht.

In noch einer weiteren alternativen Implementierung ist der Behandlungsschritt ein Aufquell- oder Schrumpfschritt. In diesem Fall wird die mindestens eine aufgetragene Schicht (3,4) einem wässrigen oder organischen Lösungsmittel ausgesetzt. Durch Diffusion von Lösungsmittel aus der mindestens einen Schicht heraus oder Diffusion von Lösungsmittel in die mindestens eine Schicht hinein wird ein Aufquellen oder Schrumpfen der Schicht bewirkt, welches die Schicht expandiert oder kontrahiert. Mit anderen Worten wird durch die Wahl eines chemisch definierten Polymers als Bestandteil der Zusammensetzung und eines angepassten Lösungsmittels ein selektives Anreichern oder Abreichern des Lösungsmittels in der Schicht erreicht, welches die Schicht kontrahiert oder expandiert.

Der mindestens eine Behandlungsschritt kann auch dadurch erfolgen, dass die mindestens eine Schicht einer elektrischen Spannung ausgesetzt wird, welche einen Phasenübergang innerhalb der Schicht verursacht. Beispielsweise kann sich der Kristallisationsgrad durch den Behandlungsschritt ändern, so dass die Schicht sich kontrahiert oder expandiert.

Weitere Behandlungsschritte, welche das Raumvolumen einer aufgetragenen Schicht verändern können, sind dem Fachmann bekannt.

Vorzugsweise erhärtet sich die mindestens eine Schicht (3,4) während des Durchführens des mindestens einen Behandlungsschritts in Schritt b). Erhärten im Sinne dieser Offenbarung bedeutet, dass sich die Schicht verfestigt. Beispielsweise kann die Schicht nach der Erhärtung einen höheren Elastizitätsmodul aufweisen.

Wenn sich die mindestens eine Schicht während des Durchführens des mindestens einen Behandlungsschritts in Schritt b) erhärtet, dann erübrigt sich ein Zwischenschritt des Verfestigens nach dem Auftragen der Schicht und dem Schritt b). In diesem Fall wird das Krümmen des Nagels durch das gleichzeitige Verfestigen und Erhärten der mindestens einen Schicht erreicht, so dass dieses Verfahren besonders effizient ist.

In einer alternativen Implementierung erhärtet sich die mindestens eine Schicht (3,4) nach dem Auftragen in Schritt a) und vor dem Durchführen des mindestens einen Behandlungsschritts in Schritt b). Wenn sich die mindestens eine Schicht nach dem Auftragen in Schritt a) und vor dem Durchführen des mindestens einen Behandlungsschritts in Schritt b) erhärtet, dann können das Erhärten, d.h. das Sich-Verfestigen der Schicht, und das Expandieren bzw. Kontrahieren der Schicht getrennt voneinander unter optimalen Bedingungen durchgeführt werden, ohne dass Kompromisslösungen hinsichtlich optimaler Aushärtung und gewünschter Expansion bzw. Kontraktion notwendig sind.

In einer bevorzugten Implementierung liegt der Elastizitätsmodul der mindestens einen Schicht (3,4) nach dem Behandlungsschritt in Schritt b) im Bereich von 1 bis 50 kN/mm², vorzugsweise im Bereich von 10 bis 30 kN/mm², insbesondere im Bereich von 15 bis 25 kN/mm².

Vorzugsweise weist die ausgehärtete Schicht einen Elastizitätsmodul auf, welcher über dem Elastizitätsmodul des Hand- oder Fußnagels liegt. Auf diese Weise können die bei Kontraktion oder Expansion der Schicht freiwerdenden Kräfte effizient auf den Hand- oder Fußnagel übertragen werden, so dass dieser sehr wirksam korrigiert werden kann. Auf der anderen Seite sollte der Elastizitätsmodul der ausgehärteten Schicht nicht so hoch sein, dass die Schicht bei längerer Verwendung auf dem Nagel unangenehme Druckstellen oder sogar Entzündungen verursacht. Aus diesem Grund ist es bevorzugt, dass der Elastizitätsmodul unter dem Elastizitätsmodul von Stahl, also unterhalb von etwa 200 kN/mm², liegt.

In einer bevorzugten Implementierung wird in Schritt a) mindestens eine erste Schicht (3) auf den Hand- oder Fußnagel (2) aufgetragen. Danach wird mindestens eine zweite Schicht (4) auf die mindestens eine erste Schicht aufgetragen. Die mindestens eine erste Schicht (3) und die mindestens eine zweite Schicht (4) unterscheiden sich hinsichtlich ihrer chemischen Zusammensetzung.

Die mindestens eine erste Schicht (3) und die mindestens eine zweite Schicht (4) können die für die mindestens eine Schicht (3,4) oben beschriebenen Merkmale aufweisen.

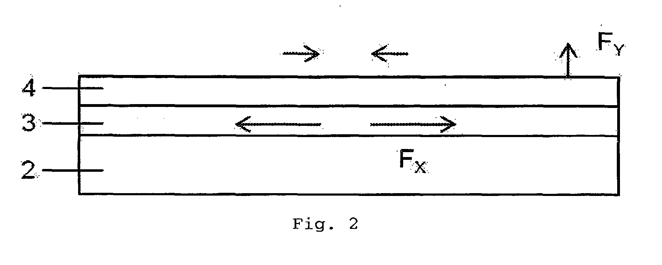

Vorzugsweise wird zuerst eine erste Schicht bestehend aus einer Zusammensetzung auf den Hand- oder Fußnagel aufgetragen und erhärtet sich. Dann wird eine zweite Schicht bestehend aus einer von der Zusammensetzung der ersten Schicht verschiedenen zweiten Zusammensetzung auf die erste Schicht aufgetragen und erhärtet sind ebenfalls. Gegebenenfalls können auf diese Weise weitere Schichten aufgetragen werden, wobei sich für die Beschichtung des Nagels insgesamt eine Laminatstruktur mit einer Vielzahl von Schichten bildet. Vorzugsweise ist die mindestens eine zweite Schicht mit der mindestens einen ersten Schicht im Wesentlichen fest verbunden, so dass es zu einer guten Kräfteübertragung zwischen den Schichten kommt, wenn eine oder mehrere dieser Schichten sich expandieren oder sich kontrahieren. Beispielsweise kann die erste Schicht eine Polymerschicht sein, welche sich durch Verdampfen von Lösungsmitteln aushärten kann, und die zweite Schicht kann ebenfalls eine Polymerschicht sein, welche sich durch Verdampfen von Lösungsmittel aushärten kann. In diesem Fall kann der Behandlungsschritt ein Lichtbehandlungsschritt sein, bei welchem sich nur die zweite Schicht im Behandlungsschritt b) weiter vernetzt und sie sich dadurch im Verhältnis zur ersten Schicht kontrahiert. Der Behandlungsschritt kann auch darin bestehen, dass auf die zweite Schicht ein Vernetzungskatalysator gegeben wird. Dieser kann die Vernetzungsreaktion katalytisch anstoßen. Der Behandlungsschritt kann ferner ein Ultraschall-Behandlungsschritt sein. In diesem Fall kann beispielsweise die Vernetzungsreaktion durch die Bestrahlung mit Ultraschallwellen angestoßen werden. Die Vernetzung in der zweiten Schicht kann aufgrund der durch die Volumenabnahme während der Reaktion flächig auftretenden Zugkräften Fₓ (siehe Figur 2) in Richtung der Nagelmitte eine Korrektur des Nagels entgegen der ungewünschten Wölbung bewirken.

Die Verwendung von mindestens zwei Schichten mit unterschiedlicher chemischer Zusammensetzung hat den Vorteil, dass sich die Schichten insgesamt stärker krümmen können und der Nagel auf diese Weise noch wirksamer korrigiert werden kann. Je mehr Schichten verwendet werden, desto wirksamer ist die Nagelkorrektur. Auf der anderen Seite wird die Auftragung von mehr als 5 oder mehr als 10 Schichten tendenziell sehr zeitaufwendig, was häufig auf eine geringere Akzeptanz des Behandlungsverfahrens beim Benutzer stößt. Aus diesem Grund ist es bevorzugt, nicht mehr als 5 oder 10 Schichten aufzutragen.

Wenn mehr als eine Schicht (3,4) aufgetragen wird, dann weist jede der Schichten, also beispielsweise sowohl die erste Schicht (3) als auch die zweite Schicht (4), eine Dicke von vorzugsweise 100 µm bis 1 mm, besonders bevorzugt 200 µm bis 800 µm, insbesondere 300 µm bis 500 µm, auf. Die Gesamtdicke aller Schichten liegt vorzugsweise im Bereich von 500 µm bis 2 mm.

In einer bevorzugten Implementierung kontrahiert sich die mindestens eine erste Schicht (3) während des Behandlungsschritts in Schritt b).

Mit anderen Worten zieht sich nur die äußerste Schicht der mehreren Schichten auf dem Hand- oder Fußnagel in der Auftragungsebene zusammen, beispielsweise die zweite von zwei Schichten, während die inneren Schichten, beispielsweise die erste von zwei Schichten, sich entweder nicht kontrahiert oder sogar expandiert, was bevorzugt ist.

In einer bevorzugten Implementierung expandiert sich die mindestens eine zweite Schicht (4) während des Behandlungsschritts in Schritt b).

Mit anderen Worten dehnt sich die innerste Schicht bzw. die innersten Schichten im Falle von mehreren Schichten auf dem Hand- oder Fußnagel in der Auftragungsebene aus, beispielsweise die erste von zwei Schichten, während die äußerste Schicht bzw. die äußersten Schichten, beispielsweise die zweite von zwei Schichten, sich entweder nicht expandiert oder sogar kontrahiert.

In einer bevorzugten Implementierung enthält die mindestens eine erste Schicht (3) Poly(4-vinylpyridin). Die mindestens eine zweite Schicht (4) enthält Polystyrol.

Vorzugsweise wird als erste Schicht (3) eine Lösung von Poly(4-vinylpyridin) (P4VP) in Chloroform oder Toluol auf den Nagel aufgetragen. Das Auftragungsverfahren ist dabei wie oben beschrieben. Nach dem Verdunsten des Lösungsmittels wird auf die erste Schicht eine Lösung von Polystyrol (PS) in Chloroform oder Toluol aufgetragen. Auch diese zweite Schicht wird dann durch Verdunsten des Lösungsmittels verfestigt. Der Behandlungsschritt umfasst danach ein Kontaktieren der Schichten mit einer wässrigen Lösung. Die wässrige Lösung ist vorzugsweise eine Lösung von Dodecylbenzolsulfonsäure (DBSA) in Wasser, besonders bevorzugt 0,1 bis 100 Gew.-% DBSA in Wasser, besonders bevorzugt 0,5 bis 10 Gew.-% DBSA in Wasser, beispielsweise 2 Gew.-% DBSA in Wasser. Vorzugsweise wird der Hand- oder Fußnagel vollständig über einen längeren Zeitraum, vorzugsweise mindestens mehrere Minuten bis mehrere Stunden in besonders schweren Fällen, in die wässrige Lösung eingetaucht.

Ohne an eine Theorie gebunden zu sein wird angenommen, dass durch den Behandlungsschritt supramolekulare Komplexe mit dem Pyridinring des P4VP gebildet werden, was zu einem höheren spezifischen Volumen der Polymere in der ersten Schicht führt. Da die Schicht aus Polystyrol in der Regel bei Kontaktierung mit der wässrigen Lösung im Wesentlichen weder expandiert noch kontrahiert, resultiert ein Biegemoment, welches zu einem Anheben des Nagels an dessen Rändern führt, so dass der Nagel im Hinblick auf zu starker Konvexität korrigiert wird.

In einer bevorzugten Implementierung wird in Schritt a) zusätzlich eine Schutzschicht auf die mindestens eine Schicht (3,4) aufgetragen.

Vorzugsweise wird zuerst eine erste Schicht bestehend aus einer Zusammensetzung auf den Nagel aufgetragen und erhärtet sich. Dann wird eine zweite Schicht bestehend aus einer anderen Zusammensetzung auf den Nagel aufgetragen und erhärtet sind. Als drittes wird die Schutzschicht auf die zweite Schicht aufgetragen. Die Schutzschicht kann jede beliebige Schicht sein, welche die darunterliegende Schicht oder die darunterliegenden Schichten hinsichtlich mechanischer oder chemischer Stabilität schützen kann. Vorzugsweise besteht die Schutzschicht ebenfalls aus einem Polymer oder einer Polymermischung, insbesondere aus vernetzten Polymeren bzw. Copolymeren. Die Auftragungsverfahren sind dabei wie oben beschrieben.

Es hat sich als vorteilhaft herausgestellt, die mindestens eine Schicht (3,4) unter Verwendung einer Schutzschicht vor mechanischen oder chemischen Einwirkungen zu schützen. Auf diese Weise können die Schichten über einen längeren Zeitraum vom Benutzer auf dem Nagel belassen werden, insbesondere über einen Zeitraum von mehreren Monaten, beispielsweise mindestens drei Monate oder mindestens sechs Monate, wobei die korrigierende Wirkung der Schichten auf das Nagelwachstum lange anhält. Auf diese Weise brauchen die Schichten nicht regelmäßig neu aufgetragen werden, sondern es reicht in der Regel eine Sitzung, um den Hand- oder Fußnagel über einen Zeitraum von 6 Monaten bis 2 Jahren zu korrigieren.

In einer bevorzugten Implementierung werden die Schritte a) und b) mehrmals wiederholt.
Beispielsweise können die Schritte a) und b) in dieser Reihenfolge mindestens zweimal oder mindestens dreimal oder mindestens fünfmal wiederholt werden. Wenn die Schritte a) und b) mehrmals wiederholt werden, dann ergibt sich eine gleichmäßigere Korrektur des Hand- oder Fußnagels und die Stärke der Krümmung kann individuell eingestellt werden. In der Regel erfolgt die Korrektur des Nagels umso stärker, je häufiger die Schritte a) und b) wiederholt werden.

In einer bevorzugten Implementierung umfasst das Verfahren zusätzlich einen Schritt c) des chemischen und/oder mechanischen Entfernens der Schichten (3,4) von dem Hand- oder Fußnagel (2).

Die mindestens eine Schicht (3,4) kann auf jede herkömmliche chemische und/oder mechanische Weise wieder vom Nagel entfernt werden. Beispielsweise können die Schichten mit einer Feile, mit einem Schmirgel- oder Schleifpapier oder mit einem Fräser vom Nagel abgeschliffen werden. Alternativ kann ein organisches Lösungsmittel dazu verwendet werden, die Schichten vom Nagel wegzulösen. In einer bevorzugten Implementierung werden chemische und mechanische Verfahrensschritte, beispielsweise Abfeilen und Lösen mit organischen Lösungsmitteln, abgewechselt.

Nach dem Entfernen der mindestens einen Schicht (3,4) bleibt vorzugsweise die Wölbung des Hand- oder Fußnagels korrigiert erhalten. Das Entfernen der Schichten wird vorzugsweise so durchgeführt, dass der Nagel nach dem Entfernen der Schicht insbesondere hinsichtlich seines Glanzes und seiner Oberflächenbeschaffenheit sein natürliches Aussehen wieder erhält.

In einer bevorzugten Implementierung verbleibt die mindestens eine Schicht (3,4) dauerhaft auf dem Nagel, wächst mit dem Nagel heraus und wird von vorne gemeinsam mit dem Nagel abgeschnitten.

Vorzugsweise ist die mindestens eine Schicht (3,4) im Wesentlichen farblos oder weist eine der natürlichen Tönung der Nagelplatte oder des Nagelbetts entsprechende Farbe auf.

Auf diese Weise wird während des Tragens der mindestens einen Schicht die natürliche Tönung des Hand- oder Fußnagels nachempfunden und es entsteht ein sehr ästhetisches Gesamterscheinungsbild. Die Verwendung von im Wesentlichen farblosen Schichten ist auch dahingehend bevorzugt, dass Unregelmäßigkeiten, beispielsweise ein Ablösen der Schichten oder Unverträglichkeiten des Nagels mit der aufgetragenen Schicht, frühzeitig erkannt werden können.

Zur Erreichung einer natürlichen Tönung kann die Schicht auch Pigmente enthalten, wie beispielsweise Titanoxid sowie alle in der dekorativen Kosmetik zugelassenen Pigmentfarbstoffe, sowie auf Basis von metalloxidbeschichteten Glimmerplättchen hergestellte Glanzpigmente in Betracht. Die Schicht kann auch andere Feststoffe enthalten, solange deren Gegenwart die Kontraktion bzw. Expansion der Schicht nicht stört. Beispielsweise kann die Schicht hochdisperse Kieselsäure zur Einstellung der Viskosität enthalten.

Die vorliegende Erfindung wird nun anhand einzelner Beispiele und Figuren weiter erläutert. Diese Beispiele und Figuren dienen nur zur Veranschaulichung des allgemeinen erfinderischen Konzepts, ohne dass die Beispiele und Figuren einen den Schutzumfang beschränkenden Charakter aufweisen sollen.

### [Kurze Beschreibung der Figur]

Fig. 1 zeigt ein Verfahren zur Korrektur eines menschlichen Hand- oder Fußnagels (2) unter Verwendung einer kontrahierenden Schicht (3).
Fig. 2 zeigt ein Verfahren zur Korrektur eines menschlichen Hand- oder Fußnagels (2) unter Verwendung von zwei Schichten (3,5).

### [Beispiele]

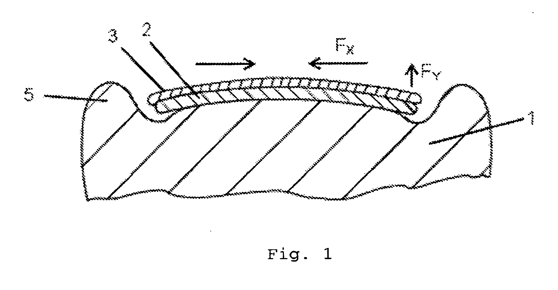

Bezug nehmend auf Fig. 1 wird ein Verfahren zur Nagelkorrektur an einem menschlichen Fuß oder an einer menschlichen Hand (1) unter Verwendung einer Schicht (3) gezeigt, wobei sich die Schicht kontrahieren kann. Die Schicht besteht aus einer Zusammensetzung, welche ein- oder mehrfach funktionelle oder funktionalisierte Urethanacrylate, partiell acrylierte Epoxydharze, wie beispielsweise Bisphenol-A-Epoxyacrylat, ein- oder mehrfach funktionelle oder funktionalisierte Acrylat-Oligomere als Vernetzer, ein oder mehrfach funktionelle oder funktionalisierte AcrylatMonomere als reaktive Verdünner und übliche UV-Initiatoren, wie beispielsweise 2-Hydroxy-2-methyl-1-methylpropan-1-on (HMPP) mit 2,4,6-Trimethylbenzoyldiphenylphosphinoxid enthält. Die Schicht wird in flüssiger Phase dünn mit einem Pinsel auf die Nagelplatte (2) aufgetragen, so dass die Schicht die Oberseite vollständig, d.h. seitlich bis zu dem Nagelfalz (4) und in der Länge von der Nagelhaut bis zu freiliegenden Nagelrand (Hyponychium), bedeckt wird. Die Dicke der Schicht beträgt ungefähr 200 bis 300 µm. Die Zusammensetzung wird dann verfestigt, indem die aufgetragene Schicht mit einer UV-Lampe (60 Watt, Wellenlänge 380 bis 450 nm) über einen Zeitraum von 1 min bestrahlt wird, wobei sich die Schicht erhärtet. Gleichzeitig kontrahiert sich die aufgetragene Schicht, so dass Kräfte F_{X} in Richtung der Nagelplattenmitte wirken. Die Nagelplatte selbst bleibt unter den Bedingungen des Behandlungsschrittes hinsichtlich der Dimensionierung im Wesentlichen konstant, d.h. kontrahiert oder expandiert sich nicht. Hierdurch entsteht eine Vertikalkraft F_{Y} auf den Rand der Nagelplatte entgegen der Rollung bzw. Wölbung, so dass eine Korrektur des Nagels erreicht wird.

Bezug nehmend auf Fig. 2 wird ein Verfahren zur Korrektur eines menschlichen Hand- oder Fußnagels (2) unter Verwendung von zwei Schichten (3, 5) gezeigt. Die innere Schicht (5) ist eine Schicht aus Poly(4-vinylpyridin) (P4VP), welche sich expandieren kann und die äußere Schicht (3) ist eine Schicht aus Polystyrol (PS), welche sich im Wesentlichen nicht verändert. Die Poly(4-vinylpyridin)- und die Polystyrol-Schichten werden aus einer Lösung in Chloroform oder Toluol durch Tauchen, Tupfen oder Bepinseln auf den Hand- bzw. Fußnagel aufgetragen. Die Dicke der Schichten (gemessen durch Ellipsometrie) beträgt circa 200 µm sowohl für die Poly(4-vinylpyridin)-Schicht als auch die Polystyrol-Schicht. Die Schichten wurden durch Bestrahlen mit kurzwelligem UV-Licht (lambda = 254 nm, Bestrahlungsdosis W = 4 J cm⁻²) unter Verwendung einer Photomaske zum Schutz der Hauptbestandteile gehärtet. UV-Licht des kurzwelligen Diapasons führt zu einer Vernetzung der beiden Polymere (siehe S. Timoshenko, J. Opt. Soc. Am. 1925, 11, 233). Nach Aushärtung der Schichten wurde der Hand- bzw. Fußnagel in eine Lösung' von 2 Gew.-% Dodecylbenzolsulfonsäure (DBSA) in Wasser getaucht, wodurch sich supramolekulare Komplexe mit den Pyridinringen von P4VP bilden. Hierdurch wird das spezifische Volumen des Polymers in der inneren Schicht erhöht. Da die Polystyrol-Schicht sich in dieser Lösung neutral verhält, d.h. insbesondere nicht die gleiche Expansion zeigt wie die P4VP-Schicht, tritt eine Wölbung entgegen der ungewünschten Wölbung des Nagels ein.

## Patentansprüche

1. Verfahren zur Korrektur eines Hand- oder Fußnagels (2), umfassend mindestens folgende Schritte:
a) Auftragen mindestens einer Schicht (3,4) mindestens einer Zusammensetzung auf einen Hand- oder Fußnagel, und
b) Durchführen mindestens eines Behandlungsschritts, in welchem sich die mindestens eine Schicht kontrahiert oder expandiert, wobei sich der Wölbungsgrad des Hand- oder Fußnagels (2) ändert und so der Hand- oder Fußnagel korrigiert wird.

2. Verfahren gemäß Anspruch 1, wobei der mindestens eine Behandlungsschritt in Schritt b) ein Licht- oder Wärmebehandlungsschritt ist.

3. Verfahren gemäß einem der Ansprüche 1 oder 2, wobei sich die mindestens eine Schicht (3,4) während des Durchführens des mindestens einen Behandlungsschritts in Schritt b) erhärtet.

4. Verfahren gemäß einem der Ansprüche 1 oder 2, wobei sich die mindestens eine Schicht (3,4) nach dem Auftragen in Schritt a) und vor dem Durchführen des mindestens einen Behandlungsschritts in Schritt b) erhärtet.

5. Verfahren gemäß einem der vorherigen Ansprüche, wobei der Elastizitätsmodul der mindestens einen Schicht (3,4) nach dem Durchführen des mindestens einen Behandlungsschritts in Schritt b) im Bereich von 1 bis 50 kN/mm², vorzugsweise im Bereich von 10 bis 30 kN/mm², insbesondere im Bereich von 15 bis 25 kN/mm² liegt.

6. Verfahren gemäß einem der vorherigen Ansprüche, wobei in Schritt a) mindestens eine erste Schicht (3) auf den Hand- oder Fußnagel (2) aufgetragen wird und auf die mindestens eine erste Schicht (3) mindestens eine zweite Schicht (4) aufgetragen wird, wobei sich die mindestens eine erste Schicht (3) und die mindestens eine zweite Schicht (4) hinsichtlich ihrer chemischen Zusammensetzungen unterscheiden.

7. Verfahren gemäß Anspruch 6, wobei sich die mindestens eine erste Schicht (3) während des Behandlungsschritts in Schritt b) kontrahiert.

8. Verfahren gemäß einem der Ansprüche 6 oder 7, wobei sich die mindestens eine zweite Schicht (4) während des Behandlungsschritts in Schritt b) expandiert.

9. Verfahren gemäß einem der Ansprüche 6 bis 8, wobei die mindestens eine erste Schicht (3) im Wesentlichen aus Poly(4-vinylpyridin) besteht und wobei die mindestens eine zweite Schicht (4) im Wesentlichen aus Polystyrol besteht.

10. Verfahren gemäß einem der vorherigen Ansprüche, wobei in Schritt a) nach dem Auftragen der mindestens einen Schicht (3,4) zusätzlich eine Schutzschicht auf die mindestens eine Schicht (3,4) aufgetragen wird.

11. Verfahren gemäß einem der vorherigen Ansprüche, wobei die Schritte a) und b) mehrmals wiederholt werden.

12. Verfahren gemäß einem der vorherigen Ansprüche, zusätzlich umfassend folgenden Schritt:
c) chemisches oder mechanisches Entfernen der Schichten von dem Hand- oder Fußnagel (2).

13. Verfahren gemäß einem der vorherigen Ansprüche, wobei die mindestens eine Schicht (3,4) im Wesentlichen farblos ist oder eine der natürlichen Tönung der Nagelplatte oder des Nagelbetts entsprechende Farbe aufweist.

## Claims

1. A method for correcting a fingernail or toenail (2), comprising at least the following steps:
a) applying of at least one layer (3,4) of at least one compound onto a fingernail or toenail, and
b) performing of at least one treatment step, in which the at least one layer contracts or expands, wherein the degree of curvature of the fingernail or toenail (2) changes and the fingernail or toenail will thus be corrected.

2. The method according to claim 1, whereby the at least one treatment step in step b) is a light or thermal treatment step.

3. The method according to any one of the claims 1 or 2, whereby the at least one layer (3,4) is hardened during the performance of the at least one treatment step in step b).

4. The method according to any one of the claims 1 or 2, whereby the at least one layer (3,4) is hardened after the application in step a) and before the performance of the at least one treatment step in step b).

5. The method according to any one of the previous claims, whereby the modulus of elasticity of the at least one layer (3,4) lies in the range from 1 to 50 kN/mm², preferably in the range from 10 to 30 kN/mm², in particular in the range from 15 to 25 kN/mm² after the performance of the at least one treatment step in step b).

6. The method according to any one of the previous claims, whereby in step a) at least one first layer (3) is applied onto the fingernail or toenail (2), and at least one second layer (4) is applied onto the at least one first layer (3), wherein the at least one first layer (3) and the at least one second layer (4) are different with regard to their chemical composition.

7. The method according to claim 6, whereby the at least one first layer (3) contracts during the treatment step in step b).

8. The method according to any one of the claims 6 or 7, whereby the at least one second layer (4) expands during the treatment step in step b).

9. The method according to any one of the claims 6 to 8, wherein the at least one first layer (3) is primarily composed of poly(4-vinyl pyridine), and wherein the at least one second layer (4) is primarily composed of polystyrene.

10. The method according to any one of the previous claims, whereby in step a), after the application of the at least one layer (3,4), a protective layer is additionally applied onto the at least one layer (3,4).

11. The method according to any one of the previous claims, whereby the steps a) and b) are repeated multiple times.

12. The method according to any one of the previous claims, comprising the following step in addition:
c) chemically or mechanically removing of the layer from the fingernail or toenail (2).

13. The method according to any one of the previous claims, wherein the at least one layer (3,4) is substantially colorless or exhibits a color corresponding to the natural shade of the nail plate or of the nail bed.

## Revendications

1. Procédé de correction d'un ongle (2) de main ou de pied, comprenant au moins les étapes suivantes :
a) application d'au moins une couche (3, 4) d'au moins une composition sur un ongle de main ou de pied, et
b) exécution d'au moins une phase de traitement où la ou les couches se contractent ou s'expansent, le galbe de l'ongle (2) de main ou de pied étant modifié et l'ongle de main ou de pied étant ainsi corrigé.

2. Procédé selon la revendication 1, où la ou les phases de traitement de l'étape b) sont une, ou des phases de traitement par la lumière ou la chaleur.

3. Procédé selon la revendication 1 ou la revendication 2, où la ou les couches (3, 4) durcissent pendant l'exécution de la ou des phases de traitement en étape b).

4. Procédé selon la revendication 1 ou la revendication 2, où la ou les couches (3, 4) durcissent après l'application en étape a) et avant l'exécution de la ou des phases de traitement en étape b).

5. Procédé selon l'une des revendications précédentes, où après l'exécution de la ou des phases de traitement de l'étape b), le module d'élasticité de la ou des couches (3, 4) est compris entre 1 et 50 kN/mm², préférentiellement entre 10 bis 30 kN/mm², tout particulièrement entre 15 et 25 kN/mm².

6. Procédé selon l'une des revendications précédentes, où en étape a) au moins une première couche (3) est appliquée sur l'ongle (2) de main ou de pied et au moins une deuxième couche (4) est appliquée sur la ou les premières couches (3), où la ou les premières couches (3) et la ou les deuxièmes couches (4) se distinguant par leur composition chimique.

7. Procédé selon la revendication 6, où la ou les premières couches (3) se contractent pendant la phase de traitement en étape b).

8. Procédé selon la revendication 6 ou la revendication 7, où la ou les deuxièmes couches (4) s'expansent pendant la phase de traitement en étape b).

9. Procédé selon l'une des revendications 6 à 8, où la ou les premières couches (3) sont essentiellement constituées de poly(4-vinyle pyridine) et où la ou les deuxièmes couches (4) sont essentiellement constituées de polystyrène.

10. Procédé selon l'une des revendications précédentes, où après l'application de la ou des couches (3, 4) en étape a), une couche protectrice est en outre appliquée sur la ou les couches (3, 4).

11. Procédé selon l'une des revendications précédentes, où les étapes a) et b) sont répétées plusieurs fois.

12. Procédé selon l'une des revendications précédentes, comprenant en outre l'étape suivante :
c) retrait chimique ou mécanique des couches de l'ongle (2) de main ou de pied.

13. Procédé selon l'une des revendications précédentes, où la ou les couches (3, 4) sont sensiblement incolores ou présentent une couleur correspondant à la coloration naturelle de la plaque cornée ou du lit de l'ongle.
